(19) Europäisches Patentamt / European Patent Office / Office européen des brevets

(11) **EP 4 031 523 B1**

(12) **EUROPEAN PATENT SPECIFICATION**

(45) Date of publication and mention
of the grant of the patent:
**19.06.2024   Bulletin 2024/25**

(21) Application number: **20768365.7**

(22) Date of filing: **15.09.2020**

(51) International Patent Classification (IPC):
**C07C 227/42** *(2006.01)*       **C07C 229/52** *(2006.01)*

(52) Cooperative Patent Classification (CPC):
(C-Sets available)
**C07C 227/42**                           (Cont.)

(86) International application number:
**PCT/EP2020/075727**

(87) International publication number:
**WO 2021/052942 (25.03.2021 Gazette 2021/12)**

(54) **SOLIDIFICATION OF HEXYL 2-[4-(DIETHYLAMINO)-2-HYDROXYBENZOYL]BENZOATE**

VERFESTIGUNG VON HEXYL-2-[4-(DIETHYLAMINO)-2-HYDROXYBENZOYL]BENZOAT

SOLIDIFICATION DE HÉXYL 2-[4-(DIÉTHYLAMINO)-2-HYDROXYBENZOYL]BENZOATE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **17.09.2019   EP 19197776**

(43) Date of publication of application:
**27.07.2022   Bulletin 2022/30**

(73) Proprietor: **BASF SE**
**67056 Ludwigshafen am Rhein (DE)**

(72) Inventors:
• **GROPP, Stefan**
**67056 Ludwigshafen (DE)**
• **WLOCH, Sebastian**
**67056 Ludwigshafen (DE)**

• **GUENTHERBERG, Norbert**
**67346 Speyer (DE)**

(74) Representative: **BASF IP Association**
**BASF SE**
**GBI-C006**
**67056 Ludwigshafen (DE)**

(56) References cited:
**EP-A1- 1 216 114      WO-A1-2008/135360
US-A- 5 324 816**

(52) Cooperative Patent Classification (CPC): (Cont.)

C-Sets
**C07C 227/42, C07C 229/52**

**Description**

The present invention relates to a process for the solidification of hexyl 2-[4-(diethylamino)-2-hydroxybenzoyl] benzoate (INCI diethylamino hydroxybenzoyl hexyl benzoate, DHHB), wherein the process comprises a step (a) of applying a shear rate of at least 400 s$^{-1}$ to liquid hexyl 2-[4-(diethylamino)-2-hydroxybenzoyl]benzoate.

Hexyl 2-[4-(diethylamino)-2-hydroxybenzoyl]benzoate (INCI diethylamino hydroxybenzoyl hexyl benzoate), also referred to as DHHB, is an UV-A filter belonging to the group of benzophenone derivatives. It is sold under the tradename Uvinul A Plus by BASF. It has a melting point of ~54 °C.

It is known in the art that solvent-free DHHB melt is hard to crystallize. An undercooled melt can stay in a metastable liquid state for weeks, until it finally crystallizes. An economical solidification process like flaking or pastillation on a cooling belt is not possible with such a very slowly crystallizing product. Only methods like crystallizing DHHB in tubs or drums and afterwards crushing it are applicable. However, these methods have a bad space time yield and lead to non-uniform particle shapes and sizes, which can lead to drawbacks, e.g., regarding its caking properties. WO2008/135360 describes a process for the crystallization of DHHB.

It was therefore an object of the present invention to provide an improved process for the solidification of DHHB. In particular, it was an object of the present invention to provide a process, which has economic advantages in comparison to the prior art methods. Moreover, it was an object to provide the solidified DHHB in uniform particle shapes and sizes, preferably exhibiting a good flowability.

It has surprisingly been found that at least one of the afore-mentioned objects can be achieved by the process of the present invention. In particular, the present invention relates to a process for the solidification of hexyl 2-[4-(diethylamino)-2-hydroxybenzoyl]benzoate (INCI diethylamino hydroxybenzoyl hexyl benzoate, DHHB), wherein the process comprises a step (a) of applying a shear rate of at least 400 s$^{-1}$ to liquid hexyl 2-[4-(diethylamino)-2-hydroxybenzoyl] benzoate.

It has surprisingly been found by the inventors of the present invention that applying a shear rate to a DHHB melt or subcooled melt can provoke a crystallization of DHHB, and therefore strongly accelerate the crystallization.

In a preferred embodiment, the melt has a temperature from more than 54 to 70 °C, preferably from more than 54 to 65 °C or the subcooled melt has a temperature from 15 to 54 °C, preferably from 20 to 35 °C.

In a preferred embodiment, the applied shear rate is at least 500 s$^{-1}$, preferably at least 1000 s$^{-1}$ or at least 2000 s$^{-1}$. An apparatus, which generates very high shear rates, is an extruder.

In a preferred embodiment, the process of the present invention is therefore performed in an extruder, a scraped surface heat exchanger, a cooling disc crystallizer, or a stirred vessel with scraping agitator.

According to one preferred embodiment, the step (a) is performed in an extruder and the process of the present invention comprises in a preferred embodiment the steps of

(a1) heating hexyl 2-[4-(diethylamino)-2-hydroxybenzoyl]benzoate until a liquid melt is obtained;
(a2) feeding the liquid hexyl 2-[4-(diethylamino)-2-hydroxybenzoyl]benzoate into an extruder;
(a3) extruding the liquid hexyl 2-[4-(diethylamino)-2-hydroxybenzoyl]benzoate.

In another preferred embodiment, the step (a) is performed in a scraped surface heat exchanger, a cooling disc crystallizer, or a stirred vessel with scraping agitator and the process of the present invention comprises the steps of

(a1 r) heating hexyl 2-[4-(diethylamino)-2-hydroxybenzoyl]benzoate until a liquid melt is obtained;
(a2r) cooling the liquid hexyl 2-[4-(diethylamino)-2-hydroxybenzoyl]benzoate to obtain a subcooled melt of hexyl 2-[4-(diethylamino)-2-hydroxybenzoyl]benzoate;
(a3r) feeding the subcooled melt of hexyl 2-[4-(diethylamino)-2-hydroxybenzoyl]benzoate into an apparatus, preferably a scraped surface heat exchanger, a cooling disc crystallizer, or a stirred vessel with scraping agitator; and
(a4r) applying shearing on the subcooled melt of hexyl 2-[4-(diethylamino)-2-hydroxybenzoyl]benzoate.

In the following, preferred embodiments regarding the process of the invention are provided. It is to be understood that the preferred embodiments mentioned above and those still to be illustrated below are preferred alone as well as in combination with each other.

As indicated above, the process of the invention comprises a step (a) of applying a shear rate of at least 400 s$^{-1}$ to liquid hexyl 2-[4-(diethylamino)-2-hydroxybenzoyl]benzoate, in order to provoke crystallization.

In a preferred embodiment, the liquid hexyl 2-[4-(diethylamino)-2-hydroxybenzoyl]benzoate is provided as a melt or a subcooled melt. It is to be understood that depending on the used apparatus the liquid hexyl 2-[4-(diethylamino)-2-hydroxybenzoyl]benzoate is provided either as a melt or as a subcooled melt.

In a preferred embodiment, the melt has a temperature from more than 54 to 70 °C, preferably from more than 54 to 65 °C or the subcooled melt has a temperature from 15 to 54 °C, preferably from 20 to 35 °C.

The application of the shear rate is decisive for the time period until nucleation starts and initiates solidification. Preferably, the shear rate is adapted such that nucleation starts within less than 2 hours, more preferably within less than 1 hour. In a preferred embodiment, the applied shear rate is at least 500 $s^{-1}$, preferably at least 1000 $s^{-1}$ or at least 2000 $s^{-1}$. In a more preferred embodiment, the applied shear rate is at least 5000 $s^{-1}$. Preferred shear rates are in the range of from 1000 to 100000, preferably from 2000 to 50000, more preferably from 8000 to 30000. Such high shear rates further reduce the time period until nucleation starts, thereby increasing the efficiency of the solidification process.

As used herein, the term "shear rate" refers to the rate at which progressive shearing deformation is applied to the liquid DHHB. In general, the shear rate may be determined for a fluid flowing between two parallel plates, one moving at a constant speed and the other one stationary based on the following equation:

$$\gamma = v/h$$

wherein "$\gamma$" is the shear rate measured in reciprocal seconds, "v" is the velocity of the moving plate, measured in meters per second, and "h" is the distance between the two parallel plates, measured in meters. Based on this principle, the rotational speed and the dimensions of an apparatus used for the application of the shear rate predetermine the shear rate.

The solidification of DHHB may be further enhanced by applying temperatures below the melting point, i.e. temperatures of less than 54 °C. In a preferred embodiment, the step (a) of the process of the invention is performed in an apparatus, which is cooled to a temperature of less than 54 °C, preferably 40 °C or less. Particularly preferred are temperatures in the range of from 20 °C to 35 °C, e.g. about 30 °C.

A suitable apparatus for the process of the invention is an extruder. In a preferred embodiment, the process of the invention is therefore performed in an extruder. The extruder may be used for partial or complete solidification. Complete solidification may take place within minutes, preferably within 30 minutes, more preferably within 20 minutes. Suitable extruders include single screw and twin screw extruders. Preferred is a twin screw extruder with co-rotating or counter-rotating screws. The extruder may be equipped with heating means and cooling means as required. The screw speed, i.e. the rotational speed of the extruder (screw speed, measured in rpm), will be adapted in order to apply the required shear rate. A skilled person is aware that the shear rate also depends amongst other things on the screw diameter and the distance between screw and wall. In particular, a high screw speed, a high screw diameter and a small distance between screw and wall results in a high shear rate and vice versa.

As indicated above, the step (a) of the process is preferably performed in an apparatus, which is cooled to a temperature of less than 54 °C, preferably less than 40 °C. Thus, it is preferred that the barrel temperature of the extruder is less than 54 °C, preferably less than 40 °C. For this purpose, a cryostat may be used.

It is preferred that the DHHB is introduced into the extruder in liquid form. Thus, it is preferred that the DHHB is heated prior to the feeding into the extruder. Accordingly, in a preferred embodiment, the process of the invention comprises the steps of

(a1) heating hexyl 2-[4-(diethylamino)-2-hydroxybenzoyl]benzoate until a liquid melt is obtained;
(a2) feeding the liquid hexyl 2-[4-(diethylamino)-2-hydroxybenzoyl]benzoate into an extruder;
(a3) extruding the liquid hexyl 2-[4-(diethylamino)-2-hydroxybenzoyl]benzoate.

In one preferred embodiment, in step (a1), a temperature of more than 54 °C is applied. Preferably, the liquid melt in step (a1) is obtained at a temperature from more than 54 to 80 °C, more preferably from 60 to 75 °C. In another preferred embodiment, the step (a2) is performed under heating of the feed to a temperature of more than 54 °C. Preferably, the liquid hexyl 2-[4-(diethylamino)-2-hydroxybenzoyl]benzoate in step (a2) is fed at a temperature from more than 54 to 75 °C, more preferably from more than 54 to 70 °C, even more preferably from 54 to 65 °C. In yet another preferred embodiment, the step (a3) is performed at a barrel temperature of less than 54 °C, preferably less than 40 °C. Preferably, the liquid hexyl 2-[4-(diethylamino)-2-hydroxybenzoyl]benzoate in step (a3) is extruded at a temperature from 10 to 54 °C, more preferably from 11 to 50 °C. In another preferred embodiment, in step (a3) the liquid hexyl 2-[4-(diethylamino)-2-hydroxybenzoyl]benzoate has a temperature from 10 to 30 °C, more preferably from 12 to 25 °C, even more preferably from 14 to 22 °C, and in particular from 15 to 21 °C.

In another preferred embodiment, the step (a3) is performed at least under atmospheric pressure, preferably at pressures higher than atmospheric pressure to ensure efficient extrusion from the extrusion head.

The extrusion step (a3) may provide the DHHB in various forms.

In one preferred embodiment, the extrusion step (a3) provides the hexyl 2-[4-(diethylamino)-2-hydroxybenzoyl]benzoate in the form of solidified strands.

In another preferred embodiment, the extrusion step (a3) provides the hexyl 2-[4-(diethylamino)-2-hydroxybenzoyl]benzoate in the form of a suspension, which is solidified by a further step of
(a4i) cooling the suspension on a cooling belt or on a drum flaker at a temperature of less than 54 °C, preferably less than 40 °C to obtain a solidified melt.

More preferably, in step (a4i), the suspension is cooled at a temperature in the range of from 20 to 30 °C, in particular at room temperature. The resulting solidified melt may then be broken into flakes or particles in further step following the step (a4i). Thus, in a preferred embodiment, the process further comprises the step of

(a5i) breaking the solidified melt into flakes or particles.

In another preferred embodiment, the extrusion step (a3) provides the hexyl 2-[4-(diethylamino)-2-hydroxybenzoyl]benzoate in the form of a suspension, which is solidified by a further step of

(a4ii) forming drops of the suspension and cooling them on a cooling belt at a temperature of 25 °C or less to obtain solidified pastilles.

In a preferred embodiment, the extruder is a single screw extruder or a twin screw extruder, preferably a twin screw extruder.

In a further preferred embodiment, the process of the invention is performed as a continuous process, wherein the liquid hexyl 2-[4-(diethylamino)-2-hydroxybenzoyl]benzoate is continuously fed into the extruder and the hexyl 2-[4-(diethylamino)-2-hydroxybenzoyl]benzoate is continuously collected from the extruder in the form of solidified strands or in the form of a suspension.

Any apparatus providing sufficient shear rate is suitable, such as a rheometer, scraped surface heat exchanger, a cooling disc crystallizer, or a stirred vessel with scraping agitator. In a preferred embodiment, the process of the invention is therefore performed in a rheometer, a scraped surface heat exchanger, a cooling disc crystallizer, or a stirred vessel with scraping agitator, more preferably a scraped surface heat exchanger, a cooling disc crystallizer, or a stirred vessel with scraping agitator.

In this connection, the liquid hexyl 2-[4-(diethylamino)-2-hydroxybenzoyl]benzoate in step (a) of the process is preferably provided as a subcooled melt.

The subcooled melt preferably has a temperature from 15 to 54 °C, more preferably from 15 to 40 °C, even more preferably from 20 to 35 °C, and in particular from 20 to 30 °C.

The apparatus preferably has a temperature from 15 to 54 °C, more preferably from 15 to 40 °C, even more preferably from 20 to 35 °C, and in particular from 20 to 30 °C.

The subcooled melt may be provided according to any known method.

When a scraped surface heat exchanger, a cooling disc crystallizer, or a stirred vessel with scraping agitator is used, it is preferred that the DHHB is introduced into the scraped surface heat exchanger, the cooling disc crystallizer, or the stirred vessel with scraping agitator as subcooled melt. Thus, it is preferred that the DHHB is first heated and then cooled prior to the feeding into the suitable apparatus, e.g. a scraped surface heat exchanger, a cooling disc crystallizer, or a stirred vessel with scraping agitator.

In a preferred embodiment, the process of the invention comprises the steps of

(a1r) heating hexyl 2-[4-(diethylamino)-2-hydroxybenzoyl]benzoate until a liquid melt is obtained;
(a2r) cooling the liquid hexyl 2-[4-(diethylamino)-2-hydroxybenzoyl]benzoate to obtain a subcooled melt of hexyl 2-[4-(diethylamino)-2-hydroxybenzoyl]benzoate;
(a3r) feeding the subcooled melt of hexyl 2-[4-(diethylamino)-2-hydroxybenzoyl]benzoate into an apparatus, preferably a scraped surface heat exchanger, a cooling disc crystallizer, or a stirred vessel with scraping agitator; and
(a4r) applying shearing on the subcooled melt of hexyl 2-[4-(diethylamino)-2-hydroxybenzoyl]benzoate.

In one preferred embodiment, in step (a1r), a temperature of more than 54 °C is applied. Preferably, the liquid melt in step (a1r) is obtained at a temperature from more than 54 to 80 °C, more preferably from 60 to 75 °C. In another preferred embodiment, in step (a2r) the subcooled melt preferably has a temperature from 15 to 54 °C, more preferably from 15 to 40 °C, even more preferably from 20 to 35 °C, and in particular from 20 to 30 °C. In another preferred embodiment, the step (a4r) is performed at a temperature of less than 54 °C, preferably less than 40 °C, more preferably less than 35 °C, and in particular less than 30 °C. In yet another preferred embodiment, the step (a4r) is performed at a temperature from 15 to 54 °C, more preferably from 15 to 40 °C, more preferably from 20 to 35 °C, and in particular from 20 to 30 °C.

In another preferred embodiment, the step (a4r) is performed at least under atmospheric pressure.

The shearing step (a4r) may provide the DHHB in various solidified forms.

The present invention is further illustrated by the following examples.

Examples

Comparative example

Solidification of DHHB was tested under the following conditions

Table 1

| Temperature | Solidification time |
|---|---|
| Room temperature | 2-3 weeks |
| Refrigerator (4 °C) | 1 week |
| Freezer (-18 °C) | 2-3 days |

The results show that the solidification kinetics for DHHB are very slow.

Example 1

Solidification of DHHB in a rheometer

Shearing was applied to a subcooled melt of DHHB having a temperature of 25 °C by a rotating cone.
Start of nucleation was determined by observing the shear rate and the viscosity. Nucleation starts when the shear rate drops and the viscosity increases.
It was observed that with increasing shear rates, the time period until nucleation started shortened. The nucleation then initiates the solidification of the DHHB. The results for different shear rates are summarized in Table 2.

Table 2

| Shear rate until nucleation [1/s] | Start of nucleation [min] | Solidification time [min] |
|---|---|---|
| 570 | 162 | 12 |
| 1000 | 102 | 10 |
| 1080 | 76 | 12 |

Example 2

Solidification of DHHB in an extruder

An extruder without a die head was used. For melting the DHHB, a heating cabinet at a temperature of 70 °C was used. The feeding unit was heated at a temperature of 60 °C. The extruder was cooled using a cryostat. The rotational speed of the screw was adapted as indicated in table 3. The throughput of the extruder and the temperature of the extrusion mass were adapted as indicated in table 3. The results for the solidification time are provided in table 3. Table 3:

| Screw speed [rpm] | Average shear rate [1/s] | Temperature of the extrusion mass [°C] | Throughput [kg/h] | Solidification time [min] |
|---|---|---|---|---|
| 100 | nd | 14 | 2 | 30 |
| 100 | nd | 8 | 1 | 10 |
| 1000 | 400 | 17 | 1 | 5 |
| 1000 | 400 | 18 | 2 | 10 |
| 1000 | 400 | 19 | 2 | 10 |

**Claims**

1. A process for the solidification of hexyl 2-[4-(diethylamino)-2-hydroxybenzoyl]benzoate (INCI diethylamino hydroxybenzoyl hexyl benzoate, DHHB), wherein the process comprises a step (a) of applying a shear rate of at least 400 $s^{-1}$ to liquid hexyl 2-[4-(diethylamino)-2-hydroxybenzoyl] benzoate.

2. The process according to claim 1, wherein the liquid hexyl 2-[4-(diethylamino)-2-hydroxybenzoyl]benzoate is provided as a melt or a subcooled melt.

3. The process according to claim 2, wherein the melt has a temperature from more than 54 to 70 °C, preferably from more than 54 to 65 °C or
   the subcooled melt has a temperature from 15 to 54 °C, preferably from 20 to 35 °C.

4. The process according to any one of claims 1 to 3, wherein the applied shear rate is at least 500 s$^{-1}$, preferably at least 1000 s$^{-1}$ or at least 2000 s$^{-1}$.

5. The process according to any one of claims 1 to 4, wherein the step (a) is performed in an apparatus, which is cooled to a temperature of less than 54 °C, preferably 40 °C or less.

6. The process according to any one of claims 1 to 5, wherein the step (a) is performed in an extruder, a scraped surface heat exchanger, a cooling disc crystallizer, or a stirred vessel with scraping agitator.

7. The process according to any one of claims 1 to 6, wherein the step (a) is performed in an extruder and wherein the process comprises the steps of

   (a1) heating hexyl 2-[4-(diethylamino)-2-hydroxybenzoyl]benzoate until a liquid melt is obtained;
   (a2) feeding the liquid hexyl 2-[4-(diethylamino)-2-hydroxybenzoyl]benzoate into an extruder;
   (a3) extruding the liquid hexyl 2-[4-(diethylamino)-2-hydroxybenzoyl]benzoate.

8. The process according to claim 7, wherein, in step (a1), a temperature of more than 54 °C is applied and/or wherein the step (a2) is performed under heating of the feed to a temperature of more than 54 °C.

9. The process according to claim 7 or 8, wherein the extrusion step (a3) provides the hexyl 2-[4-(diethylamino)-2-hydroxybenzoyl]benzoate in the form of solidified strands.

10. The process according to claim 7 or 8, wherein the extrusion step (a3) provides the hexyl 2-[4-(diethylamino)-2-hydroxybenzoyl]benzoate in the form of a suspension, which is solidified by a further step of
    (a4i) cooling the suspension on a cooling belt or on a drum flaker at a temperature of less than 54 °C, preferably less than 40 °C to obtain a solidified melt.

11. The process according to claim 10, wherein the process comprises a further step of (a5i) breaking the solidified melt into flakes or particles.

12. The process according to claim 7 or 8, wherein the extrusion step (a3) provides the hexyl 2-[4-(diethylamino)-2-hydroxybenzoyl]benzoate in the form of a suspension, which is solidified by a further step of
    (a4ii) forming drops of the suspension and cooling them on a cooling belt at a temperature of less than 54 °C, preferably less than 40 °C to obtain solidified pastilles.

13. The process according to any one of claims 7 to 12, wherein the extruder is a single screw extruder or a twin screw extruder, preferably a twin screw extruder.

14. The process according to any one of claims 7 to 13, wherein the process is performed as a continuous process, wherein the liquid hexyl 2-[4-(diethylamino)-2-hydroxybenzoyl]benzoate is continuously fed into the extruder and the hexyl 2-[4-(diethylamino)-2-hydroxybenzoyl]benzoate is continuously collected from the extruder in the form of solidified strands or in the form of a suspension.

15. The process according to any one of claims 1 to 6, wherein the step (a) is performed in a scraped surface heat exchanger, a cooling disc crystallizer, or a stirred vessel with scraping agitator and wherein the process comprises the steps of

    (a1r) heating hexyl 2-[4-(diethylamino)-2-hydroxybenzoyl]benzoate until a liquid melt is obtained;
    (a2r) cooling the liquid hexyl 2-[4-(diethylamino)-2-hydroxybenzoyl]benzoate to obtain a subcooled melt of hexyl 2-[4-(diethylamino)-2-hydroxybenzoyl]benzoate;
    (a3r) feeding the subcooled melt of hexyl 2-[4-(diethylamino)-2-hydroxybenzoyl]benzoate into an apparatus, preferably a scraped surface heat exchanger, a cooling disc crystallizer, or a stirred vessel with scraping agitator; and

(a4r) applying shearing on the subcooled melt of hexyl 2-[4-(diethylamino)-2-hydroxybenzoyl]benzoate.

**Patentansprüche**

1. Verfahren zur Verfestigung von Hexyl-2-[4-(diethylamino)-2-hydroxybenzoyl]benzoat (INCI: Diethylamino Hydroxybenzoyl Hexyl Benzoate, DHHB), wobei das Verfahren einen Schritt (a) des Anlegens einer Schergeschwindigkeit von wenigstens 400 s$^{-1}$ an flüssiges Hexyl-2-[4-(diethylamino)-2-hydroxybenzoyl]benzoat umfasst.

2. Verfahren nach Anspruch 1, wobei das flüssige Hexyl-2-[4-(diethylamino)-2-hydroxybenzoyl]benzoat als eine Schmelze oder eine unterkühlte Schmelze bereitgestellt wird.

3. Verfahren nach Anspruch 2, wobei die Schmelze eine Temperatur von mehr als 54 bis 70 °C, vorzugsweise von mehr als 54 bis 65 °C, aufweist oder
die unterkühlte Schmelze eine Temperatur von 15 bis 54 °C, vorzugsweise von 20 bis 35 °C, aufweist.

4. Verfahren nach einem der Ansprüche 1 bis 3, wobei die angelegte Schergeschwindigkeit wenigstens 500 s$^{-1}$, vorzugsweise wenigstens 1000 s$^{-1}$ oder wenigstens 2000 s$^{-1}$, beträgt.

5. Verfahren nach einem der Ansprüche 1 bis 4, wobei der Schritt (a) in einer Vorrichtung durchgeführt wird, die auf eine Temperatur von weniger als 54 °C, vorzugsweise 40 °C oder weniger, gekühlt wird.

6. Verfahren nach einem der Ansprüche 1 bis 5, wobei der Schritt (a) in einem Extruder, einem Schabewärmetauscher, einem Kühlscheibenkristallisator oder einem Rührbehälter mit Kratzrührwerk durchgeführt wird.

7. Verfahren nach einem der Ansprüche 1 bis 6, wobei der Schritt (a) in einem Extruder durchgeführt wird und wobei das Verfahren folgende Schritte umfasst:

   (a1) Erwärmen von Hexyl-2-[4-(diethylamino)-2-hydroxybenzoyl]benzoat, bis eine flüssige Schmelze erhalten wird;
   (a2) Einbringen des flüssigen Hexyl-2-[4-(diethylamino)-2-hydroxybenzoyl]benzoats in einen Extruder;
   (a3) Extrudieren des flüssigen Hexyl-2-[4-(diethylamino)-2-hydroxybenzoyl]benzoats.

8. Verfahren nach Anspruch 7, wobei in Schritt (a1) eine Temperatur von mehr als 54 °C angewendet wird und/oder wobei der Schritt (a2) unter Erwärmung des Einsatzguts auf eine Temperatur von mehr als 54 °C durchgeführt wird.

9. Verfahren nach Anspruch 7 oder 8, wobei der Extrusionsschritt (a3) das Hexyl-2-[4-(diethylamino)-2-hydroxybenzoyl]benzoat in Form verfestigter Stränge bereitstellt.

10. Verfahren nach Anspruch 7 oder 8, wobei der Extrusionsschritt (a3) das Hexyl-2-[4-(diethylamino)-2-hydroxybenzoyl]benzoat in Form einer Suspension bereitstellt, die durch folgenden weiteren Schritt verfestigt wird:
    (a4i) Kühlen der Suspension auf einem Kühlband oder auf einem Trommelflockierer bei einer Temperatur von weniger als 54 °C, vorzugsweise weniger als 40 °C, um eine verfestigte Schmelze zu erhalten.

11. Verfahren nach Anspruch 10, wobei das Verfahren folgenden weiteren Schritt umfasst:
    (a5i) Zerkleinern der verfestigten Schmelze zu Flocken oder Partikeln.

12. Verfahren nach Anspruch 7 oder 8, wobei der Extrusionsschritt (a3) das Hexyl-2-[4-(diethylamino)-2-hydroxybenzoyl]benzoat in Form einer Suspension bereitstellt, die durch folgenden weiteren Schritt verfestigt wird:
    (a4ii) Ausbilden von Tropfen der Suspension und Kühlen derselben auf einem Kühlband bei einer Temperatur von weniger als 54 °C, vorzugsweise weniger als 40 °C, um verfestigte Pastillen zu erhalten.

13. Verfahren nach einem der Ansprüche 7 bis 12, wobei der Extruder ein Einschneckenextruder oder ein Doppelschneckenextruder, vorzugsweise ein Doppelschneckenextruder, ist.

14. Verfahren nach einem der Ansprüche 7 bis 13, wobei das Verfahren als kontinuierliches Verfahren durchgeführt wird, wobei das flüssige Hexyl-2-[4-(diethylamino)-2-hydroxybenzoyl]benzoat kontinuierlich in den Extruder eingebracht wird und das Hexyl-2-[4-(diethylamino)-2-hydroxybenzoyl]benzoat kontinuierlich in Form von verfestigten Strängen oder in Form einer Suspension aus dem Extruder entnommen wird.

15. Verfahren nach einem der Ansprüche 1 bis 6, wobei der Schritt (a) in einem Schabewärmetauscher, einem Kühlscheibenkristallisator oder einem Rührbehälter mit Kratzrührwerk durchgeführt wird und wobei das Verfahren folgende Schritte umfasst:

> (alr) Erwärmen von Hexyl-2-[4-(diethylamino)-2-hydroxybenzoyl]benzoat, bis eine flüssige Schmelze erhalten wird;
> (a2r) Kühlen des flüssigen Hexyl-2-[4-(diethylamino)-2-hydroxybenzoyl]benzoats, um eine unterkühlte Schmelze von Hexyl-2-[4-(diethylamino)-2-hydroxybenzoyl]benzoat zu erhalten;
> (a3r) Einbringen der unterkühlten Schmelze von Hexyl-2-[4-(diethylamino)-2-hydroxybenzoyl]benzoat in eine Vorrichtung, vorzugsweise einen Schabewärmetauscher, einen Kühlscheibenkristallisator oder einen Rührbehälter mit Kratzrührwerk; und
> (a4r) Scheren der unterkühlten Schmelze von Hexyl-2-[4-(diethylamino)-2-hydroxybenzoyl]benzoat.

**Revendications**

1. Procédé pour la solidification du 2-[4-(diéthylamino)-2-hydroxybenzoyl]benzoate d'hexyle (INCI diethylamino hydroxybenzoyl hexyl benzoate, DHHB), dans lequel le procédé comprend une étape (a) d'application d'une vitesse de cisaillement d'au moins 400 s$^{-1}$ au 2-[4-(diéthylamino)-2-hydroxybenzoyl]benzoate d'hexyle liquide.

2. Procédé selon la revendication 1, dans lequel le 2-[4-(diéthylamino)-2-hydroxybenzoyl]benzoate d'hexyle liquide est fourni sous forme d'une masse fondue ou d'une masse fondue sous-refroidie.

3. Procédé selon la revendication 2, dans lequel la masse fondue a une température de plus de 54 à 70 °C, de préférence de plus de 54 à 65 °C ou
la masse fondue sous-refroidie a une température de 15 à 54 °C, de préférence de 20 à 35 °C.

4. Procédé selon l'une quelconque des revendications 1 à 3, dans lequel la vitesse de cisaillement appliquée est d'au moins 500 s$^{-1}$, de préférence d'au moins 1 000 s$^{-1}$ ou d'au moins 2 000 s$^{-1}$.

5. Procédé selon l'une quelconque des revendications 1 à 4, dans lequel l'étape (a) est réalisée dans un appareil qui est refroidi à une température inférieure à 54 °C, de préférence 40 °C ou moins.

6. Procédé selon l'une quelconque des revendications 1 à 5, dans lequel l'étape (a) est réalisée dans une extrudeuse, un échangeur de chaleur à surface raclée, un cristalliseur à disque de refroidissement, ou une cuve agitée avec agitateur racleur.

7. Procédé selon l'une quelconque des revendications 1 à 6, dans lequel l'étape (a) est réalisée dans une extrudeuse et dans lequel le procédé comprend les étapes de

> (a1) chauffage de 2-[4-(diéthylamino)-2-hydroxybenzoyl]benzoate d'hexyle jusqu'à l'obtention d'une masse fondue liquide ;
> (a2) alimentation du 2-[4-(diéthylamino)-2-hydroxybenzoyl]benzoate d'hexyle liquide dans une extrudeuse ;
> (a3) extrusion du 2-[4-(diéthylamino)-2-hydroxybenzoyl]benzoate d'hexyle liquide.

8. Procédé selon la revendication 7, dans lequel, à l'étape (a1), une température supérieure à 54 °C est appliquée et/ou dans lequel l'étape (a2) est réalisée sous chauffage de la charge d'alimentation jusqu'à une température supérieure à 54 °C.

9. Procédé selon la revendication 7 ou 8, dans lequel l'étape d'extrusion (a3) fournit le 2-[4-(diéthylamino)-2-hydroxybenzoyl]benzoate d'hexyle sous forme de brins solidifiés.

10. Procédé selon la revendication 7 ou 8, dans lequel l'étape d'extrusion (a3) fournit le 2-[4-(diéthylamino)-2-hydroxybenzoyl]benzoate d'hexyle sous forme d'une suspension, qui est solidifiée par une étape supplémentaire de

(a4i) refroidissement de la suspension sur une courroie de refroidissement ou sur un floconneur à tambour à une température inférieure à 54 °C, de préférence inférieure à 40 °C pour obtenir une masse fondue solidifiée.

11. Procédé selon la revendication 10, dans lequel le procédé comprend en outre une étape de
(a5i) cassure de la masse fondue solidifiée en flocons ou particules.

12. Procédé selon la revendication 7 ou 8, dans lequel l'étape d'extrusion (a3) fournit le 2-[4-(diéthylamino)-2-hydroxybenzoyl]benzoate d'hexyle sous forme d'une suspension, qui est solidifiée par une étape supplémentaire de
(a4ii) formation de gouttes de suspension et refroidissement de celles-ci sur une courroie de refroidissement à une température inférieure à 54 °C, de préférence inférieure à 40 °C pour obtenir des pastilles solidifiées.

13. Procédé selon l'une quelconque des revendications 7 à 12, dans lequel l'extrudeuse est une extrudeuse à vis simple ou une extrudeuse à vis double, de préférence une extrudeuse à vis double.

14. Procédé selon l'une quelconque des revendications 7 à 13, dans lequel le procédé est réalisé en tant que procédé continu, dans lequel le 2-[4-(diéthylamino)-2-hydroxybenzoyl]benzoate d'hexyle liquide est alimenté en continu dans l'extrudeuse et le 2-[4-(diéthylamino)-2-hydroxybenzoyl]benzoate d'hexyle est collecté en continu depuis l'extrudeuse sous forme de brins solidifiés ou sous forme d'une suspension.

15. Procédé selon l'une quelconque des revendications 1 à 6, dans lequel l'étape (a) est réalisée dans un échangeur de chaleur à surface raclée, un cristalliseur à disque de refroidissement, ou une cuve agitée avec agitateur racleur et dans lequel le procédé comprend les étapes de

(a1r) chauffage de 2-[4-(diéthylamino)-2-hydroxybenzoyl]benzoate d'hexyle jusqu'à l'obtention d'une masse fondue liquide ;
(a2r) refroidissement du 2-[4-(diéthylamino)-2-hydroxybenzoyl]benzoate d'hexyle liquide pour obtenir une masse fondue sous-refroidie de 2-[4-(diéthylamino)-2-hydroxybenzoyl]benzoate d'hexyle ;
(a3r) alimentation de la masse fondue sous-refroidie de 2-[4-(diéthylamino)-2-hydroxybenzoyl]benzoate d'hexyle dans un appareil, de préférence un échangeur de chaleur à surface raclée, un cristalliseur à disque de refroidissement, ou une cuve agitée avec agitateur racleur ; et
(a4r) application d'un cisaillement sur la masse fondue sous-refroidie de 2-[4-(diéthylamino)-2-hydroxybenzoyl]benzoate d'hexyle.

**EP 4 031 523 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008135360 A